# EUROPEAN PATENT APPLICATION

(11) **EP 4 706 661 A1**
(43) Date of publication of application: **11.03.2026**
(21) Application number: 24799842.0
(22) Date of filing: 21.03.2024
(51) Int. Cl.: A61K 31/64, A61P 25/18, A61P 25/14, A61P 25/00

(54) **NEW USE OF GLICLAZIDE**

(30) Priority: 04.05.2023 CN 202310492617
(71) Applicant: Shionogi China Co., Ltd., Shanghai 200232 (CN)
(72) Inventor: YAMAKAWA, Hidekuni, Shanghai 200232 (CN)
(74) Representative: Vossius & Partner Patentanwälte Rechtsanwälte mbB
(86) International application number: PCT/CN2024/082904
(87) International publication number: WO 2024/227384

(57) **Abstract**

The new use of gliclazide, which particularly relates to the use of gliclazide or a pharmaceutically acceptable salt thereof in the preparation of a drug for the prevention and/or treatment of psychiatric symptoms. The psychotic symptoms are generally associated with schizophrenia and other diseases for which an antipsychotic drug may be used, comprising delusions, hallucinations, thought/speech disorders, agitation, obviously disordered or abnormal movement behavior (comprising catatonia), irritability, etc.

## Description

### CROSS-REFERENCE TO RELATED APPLICATION

The present disclosure claims priority to and the benefits of Chinese patent application No. 202310492617.9 filed on May 4, 2023 and entitled "NEW USE OF GLICLAZIDE", the disclosure of which is incorporated herein by reference in its entirety.

### TECHNICAL FIELD

The present disclosure belongs to the field of medicine, and relates to new use of gliclazide, specifically to new use of gliclazide and derivatives thereof in the field of medicine.

### BACKGROUND

As presumed, at least 30 million patients worldwide suffer from psychotic symptoms (estimated on the basis of the WHO's projections).

Nowadays, there are drugs that have already been approved for treatment of schizophrenia. While atypical antipsychotics (also referred to as the second-generation antipsychotics) are useful as effective drugs for treatment of psychotic symptoms, how to strike a balance between their safety and efficacy remains a topic worthy of intensive study. In particular, a study on patients treated with antipsychotics shows that 20% or more of the cases have developed side effects - extrapyramidal symptoms (including dystonia, akathisia, parkinsonism, etc.), leading to a decrease in patients' medication adherence, which are one of the major impediments to the completion of treatment (A. Kadakia, B. L. Brady, C. Dembek, et al., The incidence and economic burden of extrapyramidal symptoms in patients with schizophrenia treated with second generation antipsychotics in a Medicaid population [J], J. Med. Econ., 2022, 25 (1): 87-98).

Gliclazide (CAS No.: 21187-98-4, molecular formula: C₁₃H₂₁N₃O₃S) is an oral hypoglycemic agent (*e.g.,* the brand name of this drug in Japan is GLIMICRON (see the Japanese Interview Form for the details); and its brand name in Europe is DIAMICRON (see the European SPC for the details)).

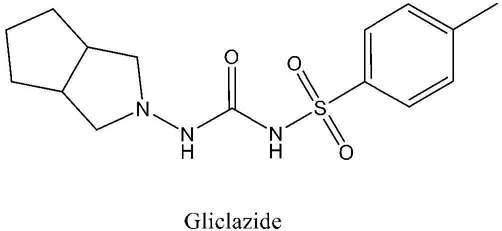

US 2022/0403469 A1 describes a method for treating schizophrenia, in which therapeutic agents are selected based on the RNA expression pattern in blood. Moreover, this patent application discloses an in silico prediction of known compounds (those with the potential for drug repurposing) potentially having therapeutic effects on hallucination symptoms according to the information of RNA expression patterns in blood of patients with the hallucination symptoms. However, the accuracy of the in silico prediction is generally limited, and it is impossible to confirm whether a drug is truly effective without actual drug administration to animals or human beings and drug evaluation. Furthermore, the one-sidedness of the in silico prediction in US 2022/0403469 A1 has been substantiated in relevant literatures, for example, clioquinol, one of the top-scoring compounds predicted in the list, is incapable of ameliorating psychotic symptoms in animal models (E.-J. Lee, H. Lee, T.-N. Huang, et al., Trans-synaptic zinc mobilization improves social interaction in two mouse models of autism through NMDAR activation [J], Nat. Commun., 2015, 6: 7168).

### SUMMARY

### Problem to Be Solved

In view of the above-mentioned technical problem existing in the prior art, the present disclosure provides medical use of gliclazide and derivatives thereof for preventing and/or treating psychotic symptom associated with schizophrenia and other diseases against which an antipsychotic agent is potentially taken.

### Solution to the Problem

### <First Aspect>

The present disclosure provides use of gliclazide or a pharmaceutically acceptable salt thereof in the manufacture of a medicament for preventing and/or treating a psychotic symptom.

Further, in the above-mentioned use, the psychotic symptom is associated with any one or more of schizophrenia and other diseases against which an antipsychotic agent is potentially taken.

Further, in the above-mentioned use, the psychotic symptom is one or more of delusions, hallucinations, disorganized thinking (speech), agitation, grossly disorganized or abnormal motor behavior (including catatonia), and irritability.

Furthermore, in the above-mentioned use, the psychotic symptom is one or more of the delusions, the hallucinations, and the agitation.

Preferably, regarding the above-mentioned psychotic symptom, the delusions include any one or more of persecutory delusion, referential delusion, somatic delusion, grandiose delusion, erotomanic delusion, nihilistic delusion, and jealous delusion.

Preferably, regarding the above-mentioned psychotic symptom, the hallucinations include any one or more of auditory hallucination, visual hallucination, gustatory hallucination, olfactory hallucination, tactile hallucination, and somatic hallucinations.

Preferably, regarding the above-mentioned psychotic symptom, the disorganized thinking (speech) includes any one or both of disorganized thinking and disorganized speech.

Preferably, regarding the above-mentioned psychotic symptom, the agitation includes any one or more of increased, often undirected, motor activity, restlessness, aggressiveness, and emotional distress.

Preferably, regarding the above-mentioned psychotic symptom, the grossly disorganized or abnormal motor behavior (including catatonia) includes any one or both of grossly disorganized or abnormal motor behavior and catatonia.

More preferably, regarding the above-mentioned grossly disorganized or abnormal motor behavior (including catatonia), the catatonia includes any one or more of negativism, mutism, stupor, and catatonic excitement.

Preferably, regarding the above-mentioned psychotic symptom, the irritability includes any one or more of annoyance, impatience, anger, sadness, quick temper, excitement, aggressiveness, and temper outbursts.

Meanwhile, the present disclosure provides use of a pharmaceutical composition or pharmaceutical formulation comprising gliclazide or a pharmaceutically acceptable salt thereof in the manufacture of a medicament for preventing and/or treating the psychotic symptom.

Further, in the above-mentioned use, the pharmaceutical composition or pharmaceutical formulation further comprises optionally one or more other active pharmaceutical ingredients and/or optionally one or more pharmaceutically acceptable excipients, in addition to the gliclazide or the pharmaceutically acceptable salt thereof.

### <Second Aspect>

The present disclosure provides gliclazide or a pharmaceutically acceptable salt thereof, for use in the prevention and/or treatment of a psychotic symptom.

Further, the psychotic symptom is associated with any one or more of schizophrenia and other diseases against which an antipsychotic agent is potentially taken.

Further, the psychotic symptom is one or more of delusions, hallucinations, disorganized thinking (speech), agitation, grossly disorganized or abnormal motor behavior (including catatonia), and irritability.

Furthermore, the psychotic symptom is one or more of the delusions, the hallucinations, and the agitation.

Preferably, regarding the above-mentioned psychotic symptom, the delusions include any one or more of persecutory delusion, referential delusion, somatic delusion, grandiose delusion, erotomanic delusion, nihilistic delusion, and jealous delusion.

Preferably, regarding the above-mentioned psychotic symptom, the hallucinations include any one or more of auditory hallucination, visual hallucination, gustatory hallucination, olfactory hallucination, tactile hallucination, and somatic hallucination.

Preferably, regarding the above-mentioned psychotic symptom, the disorganized thinking (speech) includes any one or both of disorganized thinking and disorganized speech.

Preferably, regarding the above-mentioned psychotic symptom, the agitation includes any one or more of increased, often undirected, motor activity, restlessness, aggressiveness, and emotional distress.

Preferably, regarding the above-mentioned psychotic symptom, the grossly disorganized or abnormal motor behavior (including catatonia) includes any one or both of grossly disorganized or abnormal motor behavior and catatonia.

More preferably, regarding the above-mentioned grossly disorganized or abnormal motor behavior (including catatonia), the catatonia includes any one or more of negativism, mutism, stupor, and catatonic excitement.

Preferably, regarding the above-mentioned psychotic symptom, the irritability includes any one or more of annoyance, impatience, anger, sadness, quick temper, excitement, aggressiveness, and temper outbursts.

Meanwhile, the present disclosure provides a pharmaceutical composition or pharmaceutical formulation comprising gliclazide or a pharmaceutically acceptable salt thereof, for use in the prevention and/or treatment of the psychotic symptom.

Further, the pharmaceutical composition or pharmaceutical formulation further comprises optionally one or more other active pharmaceutical ingredients and/or optionally one or more pharmaceutically acceptable excipients, in addition to the gliclazide or the pharmaceutically acceptable salt thereof.

### <Third Aspect>

The present disclosure provides a method for preventing and/or treating a psychotic symptom, comprising the step of: administering, to a subject in need thereof, a prophylactically and/or therapeutically effective amount of gliclazide or a pharmaceutically acceptable salt thereof.

Further, in the above-mentioned method, the psychotic symptom is associated with any one or more of schizophrenia and other diseases against which an antipsychotic agent is potentially taken.

Further, in the above-mentioned method, the psychotic symptom is one or more of delusions, hallucinations, disorganized thinking (speech), agitation, grossly disorganized or abnormal motor behavior (including catatonia), and irritability.

Furthermore, in the above-mentioned method, the psychotic symptom is one or more of the delusions, the hallucinations, and the agitation.

Preferably, regarding the above-mentioned psychotic symptom, the delusions include any one or more of persecutory delusion, referential delusion, somatic delusion, grandiose delusion, erotomanic delusion, nihilistic delusion, and jealous delusion.

Preferably, regarding the above-mentioned psychotic symptom, the hallucinations include any one or more of auditory hallucination, visual hallucination, gustatory hallucination, olfactory hallucination, tactile hallucination, and somatic hallucination.

Preferably, regarding the above-mentioned psychotic symptom, the disorganized thinking (speech) includes any one or both of disorganized thinking and disorganized speech.

Preferably, regarding the above-mentioned psychotic symptom, the agitation includes any one or more of increased, often undirected, motor activity, restlessness, aggressiveness, and emotional distress.

Preferably, regarding the above-mentioned psychotic symptom, the grossly disorganized or abnormal motor behavior (including catatonia) includes any one or both of grossly disorganized or abnormal motor behavior and catatonia.

More preferably, regarding the above-mentioned grossly disorganized or abnormal motor behavior (including catatonia), the catatonia includes any one or more of negativism, mutism, stupor, and catatonic excitement.

Preferably, regarding the above-mentioned psychotic symptom, the irritability includes any one or more of annoyance, impatience, anger, sadness, quick temper, excitement, aggressiveness, and temper outbursts.

Meanwhile, the present disclosure provides a method for preventing and/or treating the psychotic symptom, comprising the step of: administering a pharmaceutical composition or pharmaceutical formulation to a subject in need thereof, wherein the pharmaceutical composition or pharmaceutical formulation comprises a prophylactically and/or therapeutically effective amount of gliclazide or a pharmaceutically acceptable salt thereof.

Further, in the above-mentioned method, the pharmaceutical composition or pharmaceutical formulation further comprises optionally one or more other active pharmaceutical ingredients and/or optionally one or more pharmaceutically acceptable excipients, in addition to the gliclazide or the pharmaceutically acceptable salt thereof.

### <Fourth Aspect>

The present disclosure provides a pharmaceutical composition or pharmaceutical formulation for preventing and/or treating a psychotic symptom, comprising gliclazide or a pharmaceutically acceptable salt thereof.

Further, the psychotic symptom is associated with any one or more of schizophrenia and other diseases against which an antipsychotic agent is potentially taken.

Further, the psychotic symptom is one or more of delusions, hallucinations, disorganized thinking (speech), agitation, grossly disorganized or abnormal motor behavior (including catatonia), and irritability.

Furthermore, the psychotic symptom is one or more of the delusions, the hallucinations, and the agitation.

Preferably, regarding the above-mentioned psychotic symptom, the delusions include any one or more of persecutory delusion, referential delusion, somatic delusion, grandiose delusion, erotomanic delusion, nihilistic delusion, and jealous delusion.

Preferably, regarding the above-mentioned psychotic symptom, the hallucinations include any one or more of auditory hallucination, visual hallucination, gustatory hallucination, olfactory hallucination, tactile hallucination, and somatic hallucination.

Preferably, regarding the above-mentioned psychotic symptom, the disorganized thinking (speech) includes any one or both of disorganized thinking and disorganized speech.

Preferably, regarding the above-mentioned psychotic symptom, the agitation includes any one or more of increased, often undirected, motor activity, restlessness, aggressiveness, emotional distress, and irritability.

Preferably, regarding the above-mentioned psychotic symptom, the grossly disorganized or abnormal motor behavior (including catatonia) includes any one or both of grossly disorganized or abnormal motor behavior and catatonia.

More preferably, regarding the above-mentioned grossly disorganized or abnormal motor behavior (including catatonia), the catatonia includes any one or more of negativism, mutism, stupor, and catatonic excitement.

Preferably, regarding the above-mentioned psychotic symptom, the irritability includes any one or more of annoyance, impatience, anger, sadness, quick temper, excitement, aggressiveness, and temper outbursts.

Further, the pharmaceutical composition or pharmaceutical formulation further comprises optionally one or more other active pharmaceutical ingredients and/or optionally one or more pharmaceutically acceptable excipients, in addition to the gliclazide or the pharmaceutically acceptable salt thereof.

### Advantageous Effects

The gliclazide or derivatives thereof according to the present disclosure contribute to prevention and/or treatment of psychotic symptoms, such as hallucinations, delusions, and agitation.

### BRIEF DESCRIPTION OF THE DRAWINGS

FIG. 1 shows an inhibitory effect of gliclazide on the increased locomotor activity of mice induced by MK-801.
FIG. 2 shows an ameliorative effect of gliclazide on the pre-pulse inhibition deficits induced by MK-801.

### DETAILED DESCRIPTION OF THE EMBODIMENTS

### Definition of Terms

The exemplary examples of the present disclosure will be described in detail below. Unless otherwise specified, the term "exemplary" used herein means "used as an instance, as an example, or for illustrative purpose". An "exemplary" example given here is not necessarily construed as being superior to or better than other examples. Besides, some of the details are set forth in the specific examples below for the purpose of better explaining the present disclosure. It should be understood by a person skilled in the art that the present disclosure can still be effectively implemented even without those details.

Unless otherwise specified, either the numerical values or numerical ranges used herein should be construed as including systematic errors inevitable in industrial production; and the numerical range represented by "numerical value A to numerical value B" refers to the range including the endpoint values A and B.

Unless otherwise specified, the units used herein are all international standard units.

Unless otherwise specified, the term "may" or "can" used herein involves both the meaning of doing something and the meaning of not doing something in the subsequent processes or steps. Accordingly, the term "optional", "alternative", "optionally", or "alternatively" means that the event or circumstance described subsequently may, but not necessarily, occur, and the description involves both the case where the event or circumstance occurs and the case where the event or circumstance does not occur.

Unless otherwise specified, the phrases such as "some specific/preferred embodiments", "other specific/preferred embodiments", and "embodiments" referred to herein mean that particular elements (for example, features, structures and/or properties) described in relation to this embodiment are included in at least one of the embodiments described herein, and may or may not exist in other embodiments. Additionally, it should be understood by a person skilled in the art that the elements may be combined in any suitable manner into various embodiments.

Unless otherwise specified, the term "gliclazide or a pharmaceutically acceptable salt thereof' used herein is not limited to a specific type of isomer (stereoisomer, tautomer, etc.) thereof. In addition, the term "gliclazide or a pharmaceutically acceptable salt thereof" used herein may be replaced with an isotope-labelled compound thereof.

In addition, the term "gliclazide or a pharmaceutically acceptable salt thereof" used herein may also be administered in the form of an active metabolite or prodrug thereof.

In addition, the term "gliclazide or a pharmaceutically acceptable salt thereof' used herein may form a solvate, and the present disclosure also encompasses a variety of such solvates.

Unless otherwise specified, the term "pharmaceutically acceptable salt" or "medicinal salt" used herein means a salt suitable, within reasonable medical judgment, for use in contact with tissues of mammals (in particular humans) without undue toxicity, irritation, allergic response, etc., and commensurate with a reasonable benefit/risk ratio. For example, pharmaceutically acceptable salts of amines, carboxylic acids, and other types of compound (*e.g*., amides and ureides) are well known in the related art. The salt can be prepared *in situ* during the final separation and purification of a compound of the present disclosure, or individually prepared by reacting a free base or free acid with a suitable reagent. The pharmaceutically acceptable base addition salts of the present disclosure include, but not limited to, alkali metal salts (such as lithium salts, sodium salts, and potassium salts), alkaline earth metal salts (such as magnesium salts, calcium salts, and barium salts), transition metal salts (such as zinc salts and iron salts), and ammonium salts of gliclazide or derivatives thereof, and salts formed of gliclazide or derivatives thereof with organic bases (such as trimethylamine, triethylamine, dicyclohexylamine, ethanolamine, diethanolamine, triethanolamine, methylamine, ethylenediamine, pyridine, picoline, and quinoline) or with basic amino acids (such as lysine, arginine, and histidine). The pharmaceutically acceptable acid addition salts of the present disclosure include, but not limited to, salts formed of gliclazide or derivatives thereof with inorganic acids (such as hydrochloric acid, sulfuric acid, nitric acid, carbonic acid, hydrobromic acid, phosphoric acid, and hydroiodic acid), organic acids (such as formic acid, acetic acid, propionic acid, trifluoroacetic acid, citric acid, lactic acid, tartaric acid, oxalic acid, maleic acid, fumaric acid, succinic acid, mandelic acid, glutaric acid, malic acid, benzoic acid, phthalic acid, ascorbic acid, benzenesulfonic acid, p-toluenesulfonic acid, methanesulfonic acid, and ethanesulfonic acid) or acidic amino acids (such as glutamic acid and aspartic acid).

Unless otherwise specified, the term "solvate" or "solvated compound" used herein means a physical association of a compound of the present disclosure with one or more molecules of a solvent, whether it is organic or inorganic. The physical association involves hydrogen bonds. In some cases, for example, when one or more molecules of a solvent are accommodated in the lattice of a crystalline solid, a solvate will be able to be separated. The solvent molecules in the solvate can be arranged in a regular and/or random order. The solvate can comprise a stoichiometric or non-stoichiometric amount of solvent molecules. The term "solvate" encompasses that in the solution phase and that which can be separated. Exemplary examples of the solvate include (but not limited to) hydrates, ethanol-solvates, methanol-solvates, and isopropanol-solvates. Besides, solvation methods are well-known to a person skilled in the art.

Unless otherwise specified, the "stereoisomer" used herein includes geometric isomers, configurational isomers, conformational isomers, etc. Of these, the term "geometric isomer" (particularly "cis/trans isomer") means an isomer having a carbon-carbon or carbon-nitrogen double bond in an E or Z configuration, where the term "E" means that two substituents of higher priority are on the opposite sides of the carbon-carbon or carbon-nitrogen double bond, and the term "Z" means that the substituents of higher priority are on the same side of the carbon-carbon or carbon-nitrogen double bond. The priority order can be determined according to the Cahn-Ingold-Prelog priority rules. A compound of the present disclosure can also exist in the form of a mixture of "E" and "Z" isomers.

Unless otherwise specified, the "tautomer" used herein means isomers of different energies that can be interconverted by crossing over a low energy barrier. If tautomerism is possible (*e.g.,* in solution), a chemical equilibrium of tautomers can be reached. For example, proton tautomers (or prototropic tautomers) include the tautomers that are interconverted via proton migration, such as those formed through keto-enol tautomerism, imine-enamine tautomerism, and amide-iminol tautomerism. Valence tautomer includes the tautomers that are interconverted via rearrangement of bonding electrons.

In addition, the structural formulae described herein include all isomeric forms (*e.g.,* geometric isomeric forms, conformational isomeric forms, and tautomeric forms). Therefore, an individual stereochemical isomer of a compound of the present disclosure and a mixture of geometric isomers, conformational isomers and/or tautomers thereof are both within the scope of the present disclosure.

Unless otherwise specified, the term "isotope derivative" used herein means that a compound of the present disclosure can exist in the form of the compound with trace amounts or enriched amounts of isotopes and contain one or more atoms whose atomic weight or mass number differs from that of the most abundant atom found in nature. The isotope can be radioactive or non-radioactive. The isotopes commonly used for isotope labeling include,but not limited to, hydrogen isotopes: ²H and ³H; carbon isotopes: ¹³C and ¹⁴C; chlorine isotopes: ³⁵Cl and ³⁷Cl; fluorine isotope: ¹⁸F; iodine isotopes: ¹²³I and ¹²⁵I; nitrogen isotopes: ¹³N and ¹⁵N; oxygen isotopes: ¹⁵O, ¹⁷O, and ¹⁸O; and sulfur isotope: ³⁵S. The compounds labelled with these isotopes can be used to study the distribution of medicinal molecules in tissues. In particular, ²H and ¹³C are more widely used because they are easy to label and convenient to detect.

Unless otherwise specified, the term "active metabolite" or "active metabolic product" used herein means a physiologically active molecule resulting from metabolism of a medicament. After being metabolized in subject's body, some medicament molecules can continue to produce effects on the body. These effects are generally equivalent to or weaker than those of parent molecules, but they are still considerable. In addition, some medicaments are initially inactive and are designed to be metabolized *in vivo* to release the active pharmaceutical ingredient, and such medicaments are known as prodrugs. The medicaments are designed in such a manner probably because the prodrugs are more stable in the manufacturing and storage stages or because the prodrugs are better absorbed by the body and have better pharmacokinetic profiles.

Unless otherwise specified, the term "prodrug" used herein means a medicament transformed into the parent drug *in vivo.* The prodrugs are often useful as they can improve some confirmed, undesirable physical or biological properties. Physical properties typically pertain to the solubility (excessive or insufficient lipid solubility or water solubility) or stability. Problematic biological properties involve overquick metabolism or poor bioavailability, which themselves may be related to the physicochemical properties. For example, they can be bioavailable via oral administration, while the parent drugs cannot. The solubility of the prodrugs is also somewhat improved in the pharmaceutical compositions as compared to the parent drugs. An exemplary but non-limiting example of the prodrug may be any of the compounds of the present disclosure administered in the form of an imide or hydrazide ("prodrug") to facilitate transport across the cell membrane into the cell to be hydrolyzed into an active entity.

In addition, a stereoisomer tautomer, isotope-labelled compound, active metabolite or prodrug of gliclazide and a pharmaceutical composition or pharmaceutical formulation comprising the stereoisomer, tautomer, isotope-labelled compound, active metabolite or prodrug of gliclazide also fall within the scope of protection of the present disclosure.

In addition, a pharmaceutically acceptable salt of the stereoisomer, tautomer, isotope-labelled compound, active metabolite or prodrug of gliclazide and a pharmaceutical composition or pharmaceutical formulation comprising the pharmaceutically acceptable salt of the stereoisomer, tautomer, isotope-labelled compound, active metabolite or prodrug of gliclazide also fall within the scope of protection of the present disclosure.

In addition, a solvate of gliclazide, or the stereoisomer, tautomer, isotope-labelled compound, active metabolite or prodrug thereof, and a pharmaceutical composition or pharmaceutical formulation comprising the solvate of gliclazide, or the stereoisomer, tautomer, isotope-labelled compound, active metabolite or prodrug thereof, also fall within the scope of protection of the present disclosure.

In addition, a solvate of the pharmaceutically acceptable salt of gliclazide, or the stereoisomer, tautomer, isotope-labelled compound, active metabolite or prodrug thereof, and a pharmaceutical compositions or pharmaceutical formulations comprising the solvate of the pharmaceutically acceptable salt of gliclazide, or the stereoisomer, tautomer, isotope-labelled compound, active metabolite or prodrug thereof, also fall within the scope of protection of the present disclosure.

Unless otherwise specified, the term "active pharmaceutical ingredient (API)", "active pharmaceutical entity" or "active pharmaceutical molecule" used herein means any substance or mixture of substances for manufacturing a drug, where this substance could exhibit a pharmacological activity or other direct, relevant effects in the course of prevention, diagnosis, treatment, or symptom relief of a disease, or could affect the function or structure of a subject's body. Besides, in the present disclosure, a drug prepared from the active pharmaceutical ingredient (*e.g*., gliclazide or derivatives thereof) is useful, for example, it has the potential to become a drug with reduced side effects (*e.g*., extrapyramidal symptoms).

Unless otherwise specified, the term "pharmaceutical composition" used herein means a composition comprising a certain active pharmaceutical ingredient and optionally one or more other active pharmaceutical ingredients and/or optionally one or more pharmaceutically acceptable excipients, wherein the components in this composition may be either physically mixed or separated. Unless otherwise specified, the term "pharmaceutically acceptable excipient" has the meaning well-known to a person skilled in the art, for example, including (but not limited to) diluents (or fillers), lubricants, binders, disintegrants, stabilizers, surfactants, corrigents, odor-masking agents, solvents, cosolvents, pH regulators, osmotic pressure regulators, etc.

A pharmaceutical composition of the present disclosure is administered to a subject in need there of via any one of oral and non-oral routes of administration.

In addition, by appropriately adjusting the effective amount of the compound of the present disclosure and changing dosage form and/or various pharmaceutical additives, a pharmaceutical composition can also be prepared into a pharmaceutical formulation suitable for pediatric, geriatric, critically ill, or surgical patients.

Unless otherwise specified, the term "pharmaceutical formulation" used herein means a specific finished product of medicament prepared in accordance with a certain dosage form, which contains a certain active pharmaceutical ingredient and optionally one or more other active pharmaceutical ingredients and/or optionally one or more pharmaceutically acceptable excipients. Unless otherwise specified, the term "dosage form" has the meaning well-known to a person skilled in the art, for example, including (but not limited to) tablets, capsules, granules, lozenges, suppositories, ointments, creams, injections, suspensions, mixtures, tinctures, liniments, lotions, inhalants, aerosols, etc.

Unless otherwise stated, the term "treatment" used herein means elimination, alleviation, or amelioration of a disease or condition and/or a symptom associated therewith. While not precluded, treatment of a disease or condition does not require complete eradication of the related disease, condition, or symptom. The term "treatment" may involve administration of a therapeutically effective amount of an active pharmaceutical ingredient to a subject in need of such treatment. Treatment may be carried out aiming at a symptom, *e.g*., suppress the symptom. Treatment may be effective in a short term, or may be carried out for a medium-term period, or may be long-term treatment, *e.g*., in the case of maintenance therapy.

Unless otherwise stated, the term "prevention" used herein means avoiding the onset of a disease or condition and/or a symptom accompanied thereby in a subject or preventing a subject from acquiring a disease or condition and/or a symptom accompanied thereby, further including delaying the onset of a disease and/or a symptom accompanied thereby in a subject, and reducing the risk of a subject suffering from a disease and/or a symptom accompanied thereby. In addition, the term "prevention" further includes "prophylactic treatment", which means reducing the probability of re-development or recurrence of a disease or symptom in a subject who does not suffer from the disease or symptom but is at risk of or susceptible to the re-development of the disease or symptom or the recurrence of the disease or symptom which has been previously controlled.

Unless otherwise specified, the term "effective amount" used herein means a dose of a medicament capable of exerting an effect consistent with the prophylactic and/or therapeutic purpose or achieving a corresponding effect. For example, the "effective amount" and dosage may be set in consideration of patient's age, body weight, gender, type and severity of disease, route of administration, etc. In terms of gliclazide or derivatives thereof according to the present disclosure, a daily dosage may be, for example, from about 1 mg to about 3000 mg, or from about 1 mg to about 2000 mg, or from about 100 mg to about 2000 mg; a single dosage may be, for example, from about 1 mg to about 3000 mg, or from about 1 mg to about 2000 mg, or from about 100 mg to 2000 mg.

Unless otherwise stated, the term "psychotic symptom" used herein is defined by referring to *Diagnostic and Statistical Manual of Mental Disorders (Fifth Edition)* (referred to as DSM-V or DSM-5), which includes delusions, hallucinations, disorganized thinking (speech), agitation, grossly disorganized or abnormal motor behavior (including catatonia), and irritability, but does not include cognitive impairment.

Unless otherwise stated, the term "delusions" used herein refers to fixed beliefs that are not amenable to change in light of conflicting evidence, or refers to erroneous beliefs still held in the absence of evidence or proof. Their content may include a variety of themes (e.g., persecutory, referential, somatic, grandiose). Specifically, the persecutory delusion (*e.g*., belief that one is going to be harmed, harassed, and so forth by an individual, organization, or other group) is most common. The referential delusion (*e.g*., belief that certain gestures, comments, environmental cues, and so forth are directed at oneself) is also common. The grandiose delusion (*e.g*., when an individual believes that he or she has exceptional abilities, wealth, or fame) and the erotomanic delusion (*e.g*., when an individual believes falsely that another person is in love with him or her) are also seen. The nihilistic delusion involves the conviction that a major catastrophe will occur, and the somatic delusion focuses on the preoccupations regarding health and organ function. The jealous delusion, also known as Othello syndrome, is a pathological belief characterized by the fixed conviction that one's spouse or romantic partner is unfaithful.. Instead of first taking some measures (*e.g*., hiring a private detective or installing a surveillance camera at home to secretly record one's partner) to obtain evidence of the infidelity, a patient collects some trivial evidence (such as disheveled clothing and spotted bed sheet) and draws false inferences to prove it to be true. Delusions are deemed bizarre, if they are clearly implausible and not understandable to same-culture peers and do not derive from ordinary life experiences.

Unless otherwise stated, the term "hallucinations" used herein refers to perception-like experiences that occur without an external stimulus. They are vivid and clear, with the full force and impact of normal perceptions, and not under voluntary control. They may occur in any sensory modality (*e.g*., auditory hallucination, visual hallucination, gustatory hallucination, olfactory hallucination, tactile hallucination, or somatic hallucinations), but auditory hallucination (or phonism) is the most common in schizophrenia and related disorders. Phonism is usually experienced as voices, whether familiar or unfamiliar, that are perceived as distinct from the individual's own thoughts. Hallucinations must occur in the context of a clear sensorium; those that occur while falling asleep (hypnagogic) or waking up (hypnopompic) are considered to be within the range of normal experience.

Unless otherwise stated, the term "disorganized thinking (speech)" used herein refers to a disordered state of thinking or speech in an individual. In general, disorganized thinking (formal thought disorder) refers to illogical, unrelated, or loosely connected thoughts that can be inferred from the individual's speech. The individual may switch from one topic to another (derailment or loose associations). The answers to questions may be obliquely related or completely unrelated (tangentiality). The individual's speech may be so severely disorganized that it is nearly incomprehensible and resembles receptive aphasia in its linguistic disorganization (incoherence or "word salad"). Because mildly disorganized speech is common and nonspecific, the symptom must be severe enough to substantially impair effective communication. The severity of the impairment may be difficult to evaluate, if the person making the diagnosis comes from a different linguistic background than that of the person being examined. Less severe disorganized thinking or speech may occur during the prodromal and residual periods of schizophrenia.

Unless otherwise stated, the term "agitation" used herein refers to a behavioral syndrome characterized by increased, often undirected, motor activity, restlessness, aggressiveness, and emotional distress.

Unless otherwise stated, the term "grossly disorganized or abnormal motor behavior (including catatonia)" used herein may involve two aspects: (1) grossly disorganized or abnormal motor behavior may manifest itself in a variety of ways, ranging from childlike "silliness" to unpredictable agitation; problems may be noted in any form of goal-directed behavior, leading to difficulties in performing activities of daily living; and (2) catatonia is defined by a marked decrease in reactivity to the environment, ranging from resistance to instructions (negativism); to maintaining a rigid, inappropriate or bizarre posture; to a complete lack of verbal and motor responses (mutism and stupor); it may also include purposeless and excessive motor activity without obvious cause (catatonic excitement); other features are repeated stereotyped movements, staring, grimacing, mutism, and the echoing of speech. Although catatonia has historically been associated with schizophrenia, catatonic symptoms are nonspecific and may occur in other mental disorders (e.g., bipolar or depressive disorders with catatonia) and in medical conditions (catatonic disorder due to another medical condition).

Unless otherwise stated, the term "irritability" used herein refers to an intense but short-lasting affective disorder, including (but not limited to) annoyance, impatience, anger, sadness, quick temper, excitement, aggressiveness, and temper outbursts.

Unless otherwise stated, the term "schizophrenia" used herein is defined in the same way as "schizophrenia spectrum and other psychotic disorders" outlined in *Diagnostic and Statistical Manual of Mental Disorders (Fifth Edition)* (referred to as DSM-V or DSM-5), including schizophrenia, other psychotic disorders, and schizotypal (personality) disorders; they are defined by abnormalities in one or more of the following five domains: delusions, hallucinations, disorganized thinking (speech), grossly disorganized or abnormal motor behavior (including catatonia), and negative symptoms.

Unless otherwise stated, the term "other diseases against which an antipsychotic agent is potentially taken" used herein include, but not limited to, bipolar and related disorders, autism (autistic) spectrum disorder, depressive disorder, substance-related and addictive disorders, and dementia.

Unless otherwise stated, the term "bipolar and related disorders" used herein refers to a category of mood disorders characterized by both manic or hypomanic episodes and depressive episodes. It is generally manifested as an episodic course with mania and depression recurring or alternating, or occurring in a mixed manner. Symptoms at each episode often last for a period of time and have adverse effects on the patients' daily life, social function, etc. It includes, but is not limited to, bipolar I disorder, bipolar II disorder, cyclothymic disorder, substance/medication-induced bipolar and related disorder, bipolar and related disorder due to another medical condition, other specified bipolar and related disorder, and unspecified bipolar and related disorder.

Unless otherwise stated, the term "autism spectrum disorder" or "autistic spectrum disorder" used herein refers to persistent deficits in social communication and social interaction on a variety of occasions, including deficits in social interaction, deficits in nonverbal communication behavior in social interaction, and deficits in developing, maintaining, and comprehending interpersonal skills. In addition to deficits in social communication, the restricted, repetitive behavioral patterns, interests, or activities should also be manifested, so that the autism spectrum disorder can be diagnosed. Since symptoms vary with development and may be masked by compensatory mechanisms, diagnostic criteria may be met based on the historical information, but the current clinical manifestation should cause significant impairment.

Unless otherwise stated, the term "depressive disorder" used herein refers to a category of mood disorders mainly characterized by depressed mood, including (but not limited to) disruptive mood dysregulation disorder, major depressive disorder (including major depressive episodes), persistent depressive disorder (dysthymia), premenstrual dysphoric disorder, substance/medication-induced depressive disorder, depressive disorder due to another medical condition, other specified depressive disorder, and unspecified depressive disorder.

Unless otherwise stated, the term "substance-related and addictive disorders" used herein refers to a cluster of physiological, behavioral, and cognitive symptoms, including substance-related disorders (including, but not limited to, alcohol-related disorders, caffeine-related disorders, cannabis-related disorders, hallucinogens-related disorders, inhalants-related disorders, opioids-related disorders, sedatives, hypnotics or anxiolytics-related disorders, stimulants-related disorders, tobacco-related disorders, and other or unknown substance-related disorders) and non-substance-related disorders (including gambling disorder).

Unless otherwise stated, the term "dementia" used herein refers to a syndrome centered on acquired cognitive impairment that results in a significant decline in the patient's abilities in daily life, learning, work, and social interaction. Patient's cognitive impairment involves impairment of such abilities as memory, learning, orientation, comprehension, judgment, calculation, language, visual-spatial function, and problem analysis and solving. Dementia is described as a "neurocognitive disorder" in the DSM-5. Dementia can be classified into two categories: neurodegenerative dementia and non-neurodegenerative dementia. The former mainly includes Alzheimer's disease (AD), dementia with Lewy bodies (DLB), Parkinson's disease with dementia (PDD), frontotemporal lobar degeneration (FTLD), etc.

### Medical Use of Gliclazide

Firstly, the present disclosure provides new use of gliclazide or derivatives thereof in the field of pharmaceuticals.

To be specific, the present disclosure provides use of gliclazide or a stereoisomer, tautomer, isotope-labelled compound, active metabolite or prodrug thereof, or a pharmaceutically acceptable salt of gliclazide or the stereoisomer, tautomer, isotope-labelled compound, active metabolite or prodrug thereof, or a solvate of gliclazide or the stereoisomer, tautomer, isotope-labelled compound, active metabolite or prodrug thereof in the manufacture of a medicament for preventing and/or treating a psychotic symptom.

In some embodiments, the psychotic symptom may be associated with any one or more of schizophrenia and other diseases against which an antipsychotic agent is potentially taken.

In some specific embodiments, the psychotic symptom may be at least or merely associated with schizophrenia.

In some specific embodiments, the psychotic symptom may be at least or merely associated with other diseases against which an antipsychotic agent is potentially taken.

In some specific embodiments, the psychotic symptom may be at least or merely associated with bipolar and related disorders.

In some specific embodiments, the psychotic symptom may be at least or merely associated with autism spectrum disorder.

In some specific embodiments, the psychotic symptom may be at least or merely associated with depressive disorder.

In some specific embodiments, the psychotic symptom may be at least or merely associated with substance-related and addictive disorders.

In some specific embodiments, the psychotic symptom may be at least or merely associated with dementia (such as Alzheimer's disease or Parkinson's disease with dementia).

In some embodiments, the psychotic symptom may include a plurality of specific manifestations, for example, one or more of delusions, hallucinations, disorganized thinking (speech), agitation, grossly disorganized or abnormal motor behavior (including catatonia), and irritability.

In some specific embodiments, the psychotic symptom may include or only be the delusions.

In some more specific embodiments, the delusions may include a plurality of specific manifestations, for example, any one or more of persecutory delusion, referential delusion, somatic delusion, grandiose delusion, erotomanic delusion, nihilistic delusion, and jealous delusion.

In some more specific embodiments, the delusions may include or only be the persecutory delusion.

In some more specific embodiments, the delusions may include or only be the referential delusion.

In some more specific embodiments, the delusions may include or only be the somatic delusion.

In some more specific embodiments, the delusions may include or only be the grandiose delusion.

In some more specific embodiments, the delusions may include or only be the erotomanic delusion.

In some more specific embodiments, the delusions may include or only be the nihilistic delusion.

In some more specific embodiments, the delusions may include or only be the jealous delusion.

In some specific embodiments, the psychotic symptom may include or only be the hallucinations.

In some more specific embodiments, the hallucinations may include a plurality of specific manifestations, for example, any one or more of auditory hallucination, visual hallucination, gustatory hallucination, olfactory hallucination, tactile hallucination, and somatic hallucination.

In some more specific embodiments, the hallucinations may include or only be the auditory hallucination.

In some more specific embodiments, the hallucinations may include or only be the visual hallucination.

In some more specific embodiments, the hallucinations may include or only be the gustatory hallucination.

In some more specific embodiments, the hallucinations may include or only be the olfactory hallucination.

In some more specific embodiments, the hallucinations may include or only be the tactile hallucination.

In some more specific embodiments, the hallucinations may include or only be the somatic hallucination.

In some specific embodiments, the psychotic symptom may include or only be the disorganized thinking (speech).

In some more specific embodiments, the disorganized thinking (speech) may include a plurality of specific manifestations, for example, any one or both of disorganized thinking or disorganized speech.

In some more specific embodiments, the disorganized thinking (speech) may include or only be the disorganized thinking.

In some more specific embodiments, the disorganized thinking (speech) may include or only be the disorganized speech.

In some specific embodiments, the psychotic symptom may include or only be the agitation.

In some more specific embodiments, the agitation may include a plurality of specific manifestations, for example, any one or more of increased, often undirected, motor activity, restlessness, aggressiveness, and emotional distress.

In some more specific embodiments, the agitation may include or only be the increased, often undirected, motor activity.

In some more specific embodiments, the agitation may include or only be the restlessness.

In some more specific embodiments, the agitation may include or only be the aggressiveness.

In some more specific embodiments, the agitation may include or only be the emotional distress.

In some specific embodiments, the psychotic symptom may include or only be the grossly disorganized or abnormal motor behavior (including catatonia).

In some more specific embodiments, the grossly disorganized or abnormal motor behavior (including catatonia) may include a plurality of specific manifestations, for example, one or both of grossly disorganized or abnormal motor behavior and catatonia.

In some more specific embodiments, the grossly disorganized or abnormal motor behavior (including catatonia) may include or only be the grossly disorganized or abnormal motor behavior.

In some more specific embodiments, the grossly disorganized or abnormal motor behavior (including catatonia) may include or only be the catatonia.

In some further specific embodiments, the catatonia may include a plurality of specific manifestations, for example, any one or more of negativism, mutism, stupor, and catatonic excitement.

In some further specific embodiments, the catatonia may include or only be the negativism.

In some further specific embodiments, the catatonia may include or only be the mutism. In some further specific embodiments, the catatonia may include or only be the stupor.

In some further specific embodiments, the catatonia may include or only be the catatonic excitement.

In some specific embodiments, the psychotic symptom may include or only be the irritability.

In some more specific embodiments, the irritability may include or only be the annoyance.

In some more specific embodiments, the irritability may include or only be the impatience.

In some more specific embodiments, the irritability may include or only be the anger.

In some more specific embodiments, the irritability may include or only be the sadness.

In some more specific embodiments, the irritability may include or only be the quick temper.

In some more specific embodiments, the irritability may include or only be the excitement.

In some more specific embodiments, the irritability may include or only be the aggressiveness.

In some more specific embodiments, the irritability may include or only be the temper outbursts.

In addition to existing in a native form such as an individual compound or a mixture of individual compounds, gliclazide or derivatives thereof can further be prepared into a pharmaceutical composition or pharmaceutical formulation comprising the same (in particular a pharmaceutical composition or pharmaceutical formulation for preventing and/or treating the psychotic symptom). Therefore, the present disclosure also provides use of a pharmaceutical composition or pharmaceutical formulation comprising gliclazide or a stereoisomer, tautomer, isotope-labelled compound, active metabolite or prodrug thereof, or a pharmaceutically acceptable salt of gliclazide or the stereoisomer, tautomer, isotope-labelled compound, active metabolite or prodrug thereof, or a solvate of gliclazide or the stereoisomer, tautomer, isotope-labelled compound, active metabolite or prodrug thereof in the manufacture of a medicament for preventing and/or treating the psychotic symptom.

In some specific embodiments, the pharmaceutical composition or pharmaceutical formulation may further comprise optionally one or more other active pharmaceutical ingredients and/or optionally one or more pharmaceutically acceptable excipients.

In some more specific embodiments, the pharmaceutical composition or pharmaceutical formulation may further comprise (or exclusively comprise) optionally one or more other active pharmaceutical ingredients.

In some more specific embodiments, the pharmaceutical composition or pharmaceutical formulation may further comprise (or exclusively comprise) optionally one or more pharmaceutically acceptable excipients.

In some specific embodiments, the pharmaceutical composition or pharmaceutical formulation may further comprise (or exclusively comprise) optionally one or more other active pharmaceutical ingredients and optionally one or more pharmaceutically acceptable excipients.

In some specific embodiments, the gliclazide or the stereoisomer, tautomer, isotope-labelled compound, active metabolite or prodrug thereof, or the pharmaceutically acceptable salt of the gliclazide or the stereoisomer, tautomer, isotope-labelled compound, active metabolite or prodrug thereof, or the solvate of the gliclazide or the stereoisomer, tautomer, isotope-labelled compound, active metabolite or prodrug thereof is the sole active pharmaceutical ingredient in the medicament.

Secondly, the present disclosure provides new medical use of gliclazide or derivatives thereof.

Specifically, the present disclosure provides gliclazide or a stereoisomer, tautomer, isotope-labelled compound, active metabolite or prodrug thereof, or a pharmaceutically acceptable salt of gliclazide or the stereoisomer, tautomer, isotope-labelled compound, active metabolite or prodrug thereof, or a solvate of gliclazide or the stereoisomer, tautomer, isotope-labelled compound, active metabolite or prodrug thereof, for use in the prevention and/or treatment of a psychotic symptom.

In some specific embodiments, the psychotic symptom has the same meaning as that of the psychotic symptom described in the aforementioned new use in the field of pharmaceuticals.

In addition to existing in a native form such as an individual compound or a mixture of individual compounds, gliclazide or derivatives thereof can further be prepared into a pharmaceutical composition or pharmaceutical formulation comprising the same. Therefore, the present disclosure also provides a pharmaceutical composition or pharmaceutical formulation comprising gliclazide or a stereoisomer, tautomer, isotope-labelled compound, active metabolite or prodrug thereof, or a pharmaceutically acceptable salt of gliclazide or the stereoisomer, tautomer, isotope-labelled compound, active metabolite or prodrug thereof, or a solvate of gliclazide or the stereoisomer, tautomer, isotope-labelled compound, active metabolite or prodrug thereof, for use in the prevention and/or treatment of the psychotic symptom.

In some specific embodiments, the pharmaceutical composition or pharmaceutical formulation has the same meaning as that of the pharmaceutical composition or pharmaceutical formulation described in the aforementioned new use in the field of pharmaceuticals.

In some specific embodiments, the gliclazide or the stereoisomer, tautomer, isotope-labelled compound, active metabolite or prodrug thereof, or the pharmaceutically acceptable salt of the gliclazide or the stereoisomer, tautomer, isotope-labelled compound, active metabolite or prodrug thereof, or the solvate of the gliclazide or the stereoisomer, tautomer, isotope-labelled compound, active metabolite or prodrug thereof is used alone or acts as the sole active pharmaceutical ingredient.

Finally, the present disclosure provides new use of gliclazide or derivatives thereof in the field of methods for preventing and treating diseases.

Specifically, the present disclosure provides a method for preventing and/or treating a psychotic symptom, comprising the step of: administering, to a subject in need thereof, a prophylactically and/or therapeutically effective amount of gliclazide or a stereoisomer, tautomer, isotope-labelled compound, active metabolite or prodrug thereof, or a pharmaceutically acceptable salt of gliclazide or the stereoisomer, tautomer, isotope-labelled compound, active metabolite or prodrug thereof, or a solvate of gliclazide or the stereoisomer, tautomer, isotope-labelled compound, active metabolite or prodrug thereof.

In some specific embodiments, the subject in need thereof may include or only be vertebrates (*e.g*., fish, amphibians, reptiles, birds, and mammals).

In some more specific embodiments, the subject in need thereof may include or only be mammals (*e.g*., Marsupialia, Insectivora, Carnivora, Chiroptera, Primates, Perissodactyla, and Artiodactyla).

In some more specific embodiments, the subject in need thereof may include or only be primate species (*e.g*., Lorisidae, Lemuridae, Daubentoniidae, Tarsiidae, Cebidae, Callitrichinae, Cercopithecidae, Hylobatidae, and Hominidae).

In some further specific embodiments, the subject in need thereof may include or only be Hominidae species (*e.g.,* Pongo, Gorilla, Pan, and Homo).

In some furthermore specific embodiments, the subject in need thereof may include or only be Homo species (*e.g*., Homo sapiens).

In some specific embodiments, the psychotic symptom has the same meaning as that of the psychotic symptom described in the aforementioned new use in the field of pharmaceuticals.

In addition to existing in a native form such as an individual compound or a mixture of individual compounds, gliclazide or derivatives thereof can further be prepared into a pharmaceutical composition or pharmaceutical formulation comprising the same. Therefore, the present disclosure also provides a method for preventing and/or treating the psychotic symptom, comprising the step of: administering a pharmaceutical composition or pharmaceutical formulation to a subject in need thereof, wherein the pharmaceutical composition or pharmaceutical formulation comprises gliclazide or a stereoisomer, tautomer, isotope-labelled compound, active metabolite or prodrug thereof, or a pharmaceutically acceptable salt of gliclazide or the stereoisomer, tautomer, isotope-labelled compound, active metabolite or prodrug thereof, or a solvate of gliclazide or the stereoisomer, tautomer, isotope-labelled compound, active metabolite or prodrug thereof.

In some specific embodiments, the pharmaceutical composition or pharmaceutical formulation has the same meaning as that of the pharmaceutical composition or pharmaceutical formulation described in the aforementioned new use in the field of pharmaceuticals.

In some specific embodiments, the gliclazide or the stereoisomer, tautomer, isotope-labelled compound, active metabolite or prodrug thereof, or the pharmaceutically acceptable salt of the gliclazide or the stereoisomer, tautomer, isotope-labelled compound, active metabolite or prodrug thereof, or the solvate of the gliclazide or the stereoisomer, tautomer, isotope-labelled compound, active metabolite or prodrug thereof is administered as the sole active pharmaceutical ingredient to a subject in need thereof.

The present disclosure will be further illustrated below with reference to specific examples. Experimental methods for which no specific conditions are indicated in the following examples are generally carried out in accordance with conventional conditions or those recommended by the manufacturers. In addition, any methods and materials similar or equivalent to those described herein can be applied to the methods of the present disclosure.

### Pharmacodynamic Evaluations Using MK-801 Mouse Model

1. MK-801 model, one of the general non-clinical evaluation systems for assessing the ameliorative effects on psychotic symptom, was employed to evaluate the inhibitory effect of gliclazide on the increased locomotor activity level in mice induced by MK-801.

### 1.1 Experimental materials

### 1.1.1 Animals

7-week-old male C57BL/6J mice

Prior to the experiment, the mice were allowed to adapt themselves to the environment for one week. Each of the mice was subjected to health examination, including evaluations of the hair, limbs, and mouth, and was examined for signs of abnormal posture and movement.

### 1.1.2 Drugs

Methylcellulose;
MK-801 (or referred to as Dizocilpine);
Gliclazide.

### 1.2 Experimental steps

### 1.2.1 Formulation of drug solutions

### 1.2.1.1 Formulation of a solution of MK-801

An appropriate amount of MK-801 was weighed into a clean tube, and then an appropriate amount of saline was added thereto, sonicated and vortexed until the solution became clear. The solution had a concentration of 0.2 to 0.3 mg/mL, and stored at -20°C for subsequent use. An appropriate amount of MK-801 solution was transferred into another clean tube, and then diluted, by adding thereto an appropriate amount of saline, to prepare a solution of MK-801 at a concentration of 0.02 to 0.03 mg/mL. When administered (*e.g*., intraperitoneally injected), the dose used was 10 mL/kg.

### 1.2.1.2 Formulation of a solution of gliclazide

An appropriate amount of gliclazide was weighed into a clean tube, and then an appropriate amount of 0.5%w/v aqueous methylcellulose solution was added thereto, sonicated and vortexed until the solution became clear. Three parallel samples were set for each group, corresponding to the solutions of gliclazide prepared at the concentrations of 8 mg/mL, 16 mg/mL, and 32 mg/mL, respectively. When administered (*e.g*., orally or by gavage), the dose used was 10 mL/kg, *i.e.,* 80 mg/kg, 160 mg/kg, and 320 mg/kg for the low-, medium-, and high-dose gliclazide groups, respectively.

### 1.2.2 Dosing and monitoring

The solution of MK-801 (at a dose of 0.2 to 0.3 mg/kg body weight) was administered to mice via intraperitoneal injection to induce their activities. The increased locomotor activity level (this parameter could be used as the behavioral indicator for psychotic symptom) in mice was determined.

Mice in the negative control group and the low-, medium-, and high-dose gliclazide groups (8 mice in each group) were orally administered with the solutions of gliclazide at a dose of 0, 80, 160, and 320 mg/kg body weight, respectively, and at the time point of 60 min before administering MK-801, and the increased locomotor activity levels in mice in the groups were determined respectively in a 10-minute period between 10 min and 20 min after administering MK-801.

### 1.3 Experimental results

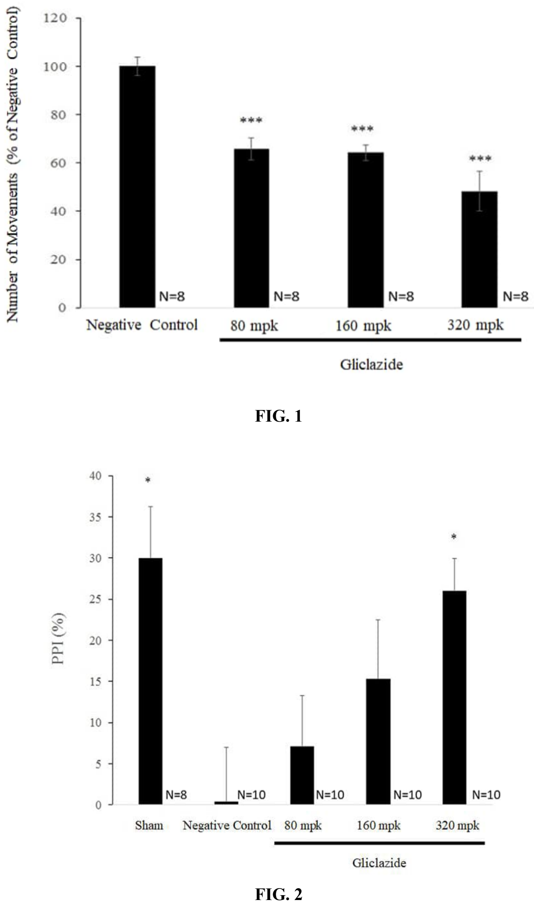

As appreciated from the experimental results shown in FIG. 1 (*** indicated p<0.001 as compared to the negative control group, assessed using the Kruskal-Wallis test followed by the Conover's test), gliclazide could significantly inhibit the increase in the locomotor activity level in mice induced by MK-801.

### 2. Ameliorative effect of gliclazide on pre-pulse inhibition deficits

The psychotic symptom associated with schizophrenia was known to be related to the cognitive function. The present disclosure verified the effect of gliclazide on the impaired sensorimotor gating in MK-801 mouse models, accordingly.

### 2.1 Experimental materials

### 2.1.1 Animals

The same as those used in Item 1.1.1.

### 2.1.2 Drugs

The same as those used in Item 1.1.2.

### 2.2 Experiment steps

### 2.2.1 Formulation of drug solutions

### 2.2.1.1 Formulation of a solution of MK-801

The same as that described in Item 1.2.1.1.

### 2.2.1.2 Formulation of a solution of gliclazide

The same as that described in Item 1.2.1.2.

### 2.2.2 Dosing and monitoring

Mice in the sham group, the negative control group, and the low-, medium-, and high-dose gliclazide groups (8 mice in the sham group, and 10 mice in the rest of four groups) were orally administered with the solutions of gliclazide at a dose of 0, 0, 80, 160, and 320 mg/kg body weight, respectively, and at the time point of 60 min before administering MK-801. Subsequently, the mice in the negative control group and the low-, medium-, and high-dose groups were intraperitoneally administered with the solutions of MK-801 (at a dose of 0.2 to 0.3 mg/kg body weight), and the mice in the sham group were intraperitoneally administered with the same volume of saline. At the time point of 20 min after dosing, the function of sensorimotor gating of mice was determined using the evaluation system for pre-pulse inhibition of the acoustic startle reflex.

### 2.2.3 Data analysis

The pre-pulse inhibition (PPI) was calculated by the following equation: (startle response with pre-pulse acoustic stimulus) / (startle response without pre-pulse acoustic stimulus) × 100%.

### 2.3 Experimental results

As appreciated from the experimental results shown in FIG. 2 (* indicated p<0.05 as compared to the negative control group), gliclazide could ameliorate the pre-pulse inhibition deficits induced by MK-801, and the ameliorative effect was dose-dependent within a certain range. In addition, the result of the high-dose gliclazide group (320 mg/kg) showed a significantly ameliorative effect as compared to the negative control group, roughly comparable to that of the sham group.

Exemplary but non-exhaustive examples of the present disclosure have been described above, and the present disclosure is not limited to the specific examples disclosed herein. A number of modifications and variations will be apparent to a person skilled in the art without departing from the scopes and spirits of the specific examples, and are all within the scope of the present disclosure.

## Claims

1. Use of gliclazide or a pharmaceutically acceptable salt thereof in the manufacture of a medicament for preventing and/or treating a psychotic symptom.

2. The use according to claim 1, wherein
the psychotic symptom is associated with any one or more of schizophrenia and other diseases against which an antipsychotic agent is potentially taken.

3. The use according to claim 1 or 2, wherein
the psychotic symptom is any one or more of delusions, hallucinations, disorganized thinking (speech), agitation, grossly disorganized or abnormal motor behavior (including catatonia), and irritability.

4. The use according to claim 3, wherein
the psychotic symptom is any one or more of the delusions, the hallucinations, and the agitation.

5. The use according to any one of claims 1 to 4, wherein
the gliclazide or the pharmaceutically acceptable salt thereof is the sole active pharmaceutical ingredient in the medicament.

6. Use of a pharmaceutical composition or pharmaceutical formulation comprising gliclazide or a pharmaceutically acceptable salt thereof in the manufacture of a medicament for preventing and/or treating a psychotic symptom, wherein the pharmaceutical composition or pharmaceutical formulation further comprises optionally one or more other active pharmaceutical ingredients and/or optionally one or more pharmaceutically acceptable excipients.

7. The use according to claim 6, wherein
the psychotic symptom is associated with any one or more of schizophrenia and other diseases against which an antipsychotic agent is potentially taken.

8. The use according to claim 6 or 7, wherein
the psychotic symptom is any one or more of delusions, hallucinations, disorganized thinking (speech), agitation, grossly disorganized or abnormal motor behavior (including catatonia), and irritability.

9. The use according to claim 8, wherein
the psychotic symptom is any one or more of the delusions, the hallucinations, and the agitation.

10. The use according to any one of claims 6 to 9, wherein
the gliclazide or the pharmaceutically acceptable salt thereof is the sole active pharmaceutical ingredient in the medicament.
